(19) Europäisches Patentamt
European Patent Office
Office européen des brevets

(11) Publication number: 0 066 827
B1

(12) # EUROPEAN PATENT SPECIFICATION

(45) Date of publication of patent specification: 22.10.86

(51) Int. Cl.⁴: C 07 D 213/64

(21) Application number: 82104766.9

(22) Date of filing: 28.05.82

(54) **Process for making pyridyloxyphenol compounds.**

(30) Priority: 01.06.81 US 269300

(43) Date of publication of application:
15.12.82 Bulletin 82/50

(45) Publication of the grant of the patent:
22.10.86 Bulletin 86/43

(84) Designated Contracting States:
BE CH DE FR GB IT LI NL SE

(56) References cited:
EP-A-0 021 613
US-A-4 046 553
US-A-4 214 086
US-A-4 266 063
US-A-4 275 212

**The file contains technical information submitted after the application was filed and not included in this specification**

(73) Proprietor: THE DOW CHEMICAL COMPANY
2030 Dow Center Abbott Road P.O. Box 1967
Midland, MI 48640 (US)

(72) Inventor: Krauss, Richard Carl
4308 Congress Drive
Midland Michigan 48640 (US)

(74) Representative: Weickmann, Heinrich, Dipl.-Ing. et al
Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820
D-8000 München 86 (DE)

EP 0 066 827 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Pyridyloxyphenol compounds may be prepared by the reaction of a 2-substituted pyridine compound with a hydroquinone and an alkaline material, as taught in U.S. Patent 4 046 553 issued September 6, 1977 and in U.S. Patent 4 275 212 issued June 23, 1981. In these reactions, the products of side reactions, such as hydrolysis, often constitute 5 or more weight percent of the obtained material. According to U.S. Patent 4 214 086 issued July 22, 1980, the desired compound may also be prepared by dissolving hydroquinone and 1.2 to 2 equivalents of pulverised potassium hydroxide in a polar aprotic solvent, heating the solvent in a nitrogen atmosphere and stirring it at elevated temperature, whereupon 0.9 to 1.0 mol of a 2-halogenopyridine are slowly added and stirring at elevated temperature is continued. In accordance with said reference, a diether is formed as an intermediate, which is then transetherified in situ to yield the desired pyridyloxyphenol. This reference also teaches that, if desired, a further solvent, which forms an azeotrope with water, may be employed.

This invention provides a process for making pyridyloxyphenol compounds by reacting a 2-substituted pyridine compound with a hydroquinone and an alkaline material under essentially anhydrous conditions.

More particularly, the invention provides a process for making pyridyloxyphenol compounds having the formula

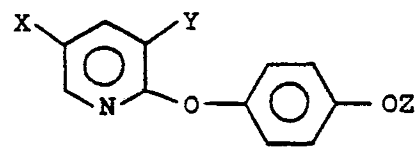

wherein X is chloro or trifluoromethyl, Y is hydrogen or chloro and Z is hydrogen, sodium or potassium, which comprises dissolving hydroquinone in a polar aprotic solvent, heating under vaccum to degas, adding sufficient aqueous sodium or potassium hydroxide to neutralize from 75 to 100 percent of the hydroquinone, while continuing to heat under vacuum to distill off the water until less than 1 weight percent water, based on total weight of solvent and reactants, remains releasing the vacuum with a dry inert gas,

adding a 2-halopyridine having the formula

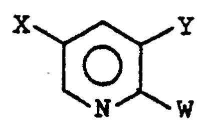

wherein X and Y are as above defined and W is bromo, chloro, fluoro or iodo, and reacting at a temperature from 25° to 150°C.

Detailed Description of the Invention

In carrying out the process of this invention, the selected pyridine and hydroquinone reactants are contacted in the absence of oxygen, i.e, under an inert atmosphere, e.g., nitrogen, helium or argon, in the presence of an inert carrier medium, i.e., a polar aprotic solvent such as, for example, dimethylsulfoxide (DMSO), dimethylformamide, diethylacetamide or N-methylpyrrolidone under essentially anhydrous conditions to provide the desired pyridyloxyphenol compounds in higher yields and purity than prior art methods, i.e., the products of side reactions such as hydrolysis often constitute less than 1 percent and advantageously less than 0.5 percent of the obtained material.

To obtain the desired anhydrous conditions the hydroquinone reactant is, typically, dissolved in the polar aprotic solvent, the solution heated under vacuum (advantageously about 100°C at 133 mbar) to degas, the desired amount of aqueous caustic is added while continuing to heat under vacuum and water is removed by distillation. When essentially all of the water is removed, i.e., less than 1 weight percent and, preferably, less than 0.5 weight percent (based on total weight of the mixture) of water remains, the vacuum is released with dry inert gas, e.g., nitrogen, the slurry of disodium or dipotassium hydroquinone is cooled and the desired pyridine compound is added. This latter reaction may be carried out at temperatures of from 25° to 150°C, but is preferably carried out at 70° to 100°C.

The amount of sodium or potassium hydroxide required is that amount necessary to neutralize from 75 to 100 percent, advantageously 90 to 100 percent, of the hydroquinone reactant.

The invention is further illustrated by the following examples in which all stated percentages are weight percents unless otherwise indicated:

Example 1

A solution of 22.5 g (0.205 mole) of hydroquinone in 200 ml of DMSO wsa heated to 100°C under vacuum (133 mbar 100 mm Hg) to degas and 31.5 g (0.394 mole) of 50 percent aqueous NaOH were added over 10 minutes while continuing to heat the solution at 133 mbar pressure. Water began to distill and was removed through a five-plate Oldershaw Column operated with reflux. The pot temperature rose gradually

to 128°C while the head temperature remained constant at about 55°C. After about 35 ml of distillate had been collected, the head temperature rose rapidly to 125°C. The distillation was continued until about 80 ml had been collected. The vacuum was released with dry $N_2$, the slurry of disodium hydroquinone was cooled to 70°C and 43.2 g (0.2 mole) of 2,3-dichloro-5-(trifluoromethyl) pyridine were added over about 5 minutes. The reaction exothermed and cooling was initially required to maintain the temperature below 80°C for 30 minutes. The reaction was held at 80°C. At this time, analysis by high pressure liquid chromatography (HPLC) showed the product composition to be:

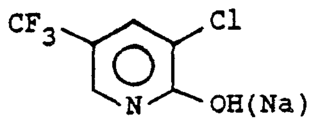

0.13 wt. %

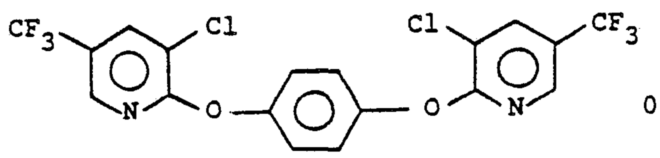

0.3 wt. %

(Bis)

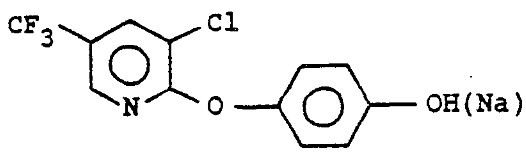

~99 wt. %

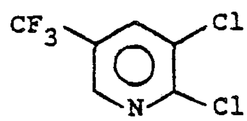

none detected

Example 2

A solution of 22.5 g (0.205 mole); of hydroquinone in 200 ml of DMSO was heated to boiling under a five-plate Oldershaw Column at 133 mbar pressure and 25.5g (0.454 mole) of KOH in 25 ml water were added dropwise over three hours while continuously distilling off water. After addition was complete, the distillation was contined to a pot temperature of 128°C and head temperature of 123°C. The total distillate was 110 ml. The vacuum was released with dry nitrogen, the slurry cooled to 70°C and 43.2 g (0.2 mole) of 2,3-dichloro-5-(trifluoromethyl)pyridine were added dropwise over 10 minutes. The reaction was held at 80°C for 90 minutes. HPLC analysis showed the desired product with only 1.1 weight percent of

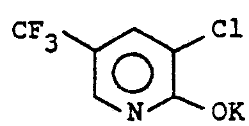

and 0.5 percent of the bis compound.

Example 3

A solution of 22.5 g (0.205 mole) of hydroquinone in 300 ml N-methylpyrrolidone was heated to boiling at 26,7 mbar pressure under a 10-plate Oldershaw Column, 31.5 g (0.394 mole) of 50 percent aqueous sodium hydroxide were added dropwise over 20 minutes and water was distilled off. The total distillate was 50 ml. The vacuum was released with dry nitrogen, the slurry cooled to 80°C and 43.2 g (0.2 mole) of 2,3-dichloro-5-(trifluoromethyl)pyridine were added all at once. The reaction mixture exothermed about 10°C and was further heated to 100°C and held one hour to give the desired product containing only 1.4 weight percent of

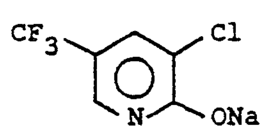

and 0.25 weight percent of the bis compound.

Example 4

A solution of 11.3 g (0.103 mole) of hydroqinone in 145 ml DMSO was heated to boiling under a

10-plate Oldershaw Column at 133 mbar pressure, 15.6 g (0.195 mole) of 50 percent aqueous sodium hydroxide were added dropwise over two minutes and water was distilled off. The total distillate was 60 ml. The vacuum was released with dry nitrogen, the slurry cooled to 70°C and 18.1 g (0.1 mole) of 2-chloro-5-(trifluoromethyl)pyridine were added all at once. The reaction exothermed to 100°C and was held there for 30 minutes to give sodium 4-(5-(trifluoromethyl)-2-pyridyloxy)phenate containing less than 1 weight percent of

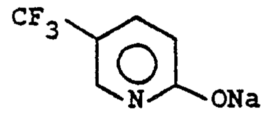

and 0.5 weight percent of the bis compound.

Example 5

Following the procedure of Example 4 a slurry of 0.1 mole hydroquinone disodium salt was prepared and 21.6 g (0.1 mole) of 2,3-dichloro-5-(trifluoromethyl)pyridine was added at 110°C. The reaction exothermed and was held at 130°C to 135°C for five minutes to give the desired product containing only 0.3 weight percent of

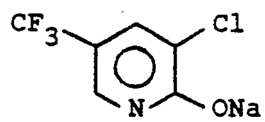

and 0.1 weight percent of the bis compound.

## Claims

1. Process for making pyridyloxyphenol compounds having the formula

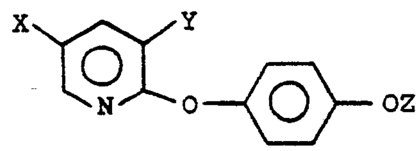

wherein X is chloro or trifluoromethyl, Y is hydrogen or chloro and Z is hydrogen, sodium or potassium, which comprises dissolving hydroquinone in a poolar aprotic solvent, heating under vacuum to degas, adding sufficient aqueous sodium or potassium hydroxide to neutralize from 75 to 100 percent of the hydroquinone, while continuing to heat under vacuum to distill off the water until less than 1 weight percent water, based on total weight of solvent and reactants, remains, releasing the vacuum with a dry inert gas adding a 2-halopyridine having the formula

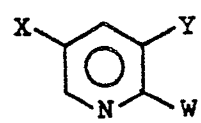

wherein X and Y are as above defined and W is bromo, chloro, fluoro or iodo and reacting at a temperature of from 25°C to 150°C.

2. Process of Claim 1 wherein the reaction temperature is from 70° to 100°C.

3. Process of Claim 1 wherein the 2-holopyridine compound is 2,3-dichloro-5-(trifluoromethyl)-pyridine.

4. Process of Claim 1 wherein the 2-halopyridine compound is 2-chloro-5-(trfluoromethyl)pyridine.

5. Process of Claim 1 wherein the solvent is dimethylsulfoxide.

6. Process of Claim 1 wherein the solvent is N-methyl pyrrolidone.

7. Process of Claim 1 wherein the hydroquinone has been from 90 to 100 percent neutralized to the sodium salt.

## Patentansprüche

1. Verfahren zur Herstellung von Pyridyloxyphenol-Verbindungen der Formel

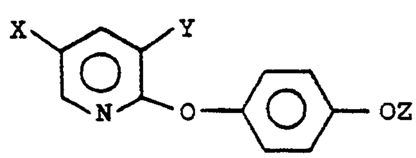

worin X Chlor oder Trifluormethyl ist, Y Wasserstoff oder Chlor ist, und Z Wasserstoff, Natrium oder Kalium

ist, dadurch gekennzeichnet, daß man Hydrochinon in einem polaren aprotischen Lösungsmittel löst, zur Entgasung unter Vakuum erhitzt, ausreichend wässriges Natrium- oder Kaliumhydroxide zufügt, um 75 bis 100% des Hydrochinons zu neutralisieren, während man das Erhitzen unter Vakuum fortsetzt, um das Wasser abzudestillieren, bis weniger als 1 Gew.% Wasser, bezogen auf das Gesamtgewicht an Lösungsmittel und Reaktanten, verbleibt, das Vakuum mit einem trockenen inerten Gas aufhebt, 2-Halogenpyridin der Formel

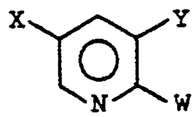

worin X und Y die oben angegebene Bedeutung besitzen, und W Brom, Chlor, Fluor oder Jod ist, hinzufügt, und die Reaktion bei einer Temperatur von 25 bis 150°C durchführt.

2. Verfahren nach Anspruch 1, dadurch gekennzeichnet, daß die Reaktionstemperatur 70 bis 100°C ist.

3. Verfahren nach Anspruch 1, dadurch gekennzeichnet, daß die 2-Halogenpyridin-Verbindung 2,3-Dichlor-5-(trifluormethyl)-pyridin ist.

4. Verfahren nach Anspruch 1, dadurch gekennzeichnet, daß die 2-Halogenpyridin-Verbindung 2-Chlor-5-(trifluormethyl)-pyridin ist.

5. Verfahren nach Anspruch 1, dadurch gekennzeichnet, daß das Lösungsmittel Dimethylsulfoxid ist.

6. Verfahren nach Anspruch 1, dadurch gekennzeichnet, daß das Lösungsmittel N-Methylpyrrolidon ist.

7. Verfahren nach Anspruch 1, dadurch gekennzeichnet, daß das Hydrochinon zu 90 bis 100% zum Natriumsalz neutralisiert wurde.

**Revendications**

1. Procédé pour la préparation de composés pyridyloxyphénol répondant à la formule

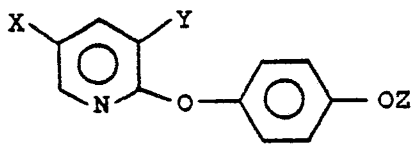

dans laquelle X est un atome de chlore ou le groupe trifluorométhyle, Y est un atome d'hydrogène ou de chlore et Z est un atome d'hydrogène, de sodium ou de potassium, caractérisé en ce qu'il comprend le fait de dissoudre l'hydroquinone dans un solvant polaire aprotique, de chauffer sous vide pour dégazer, d'ajouter une solution aqueuse d'hydroxyde de sodium ou de potassium en quantité suffisante pour neutraliser de 75 à 100 pour cent de l'hydroquinone, tout en poursuivant le chauffage sous vide pour éliminer l'eau par distillation jusqu'à ce qu'il reste moins de 1 pour cent en poids d'eau, par rapport au poids total du solvant et des corps réagissants, de rompre le vide avec un gaz inerte anhydre, d'ajouter une 2-halopyridine répondant à la formule

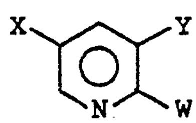

dans laquelle X et Y sont tels que définis ci-dessus et W est un atome de brome, de chlore, de fluor ou d'iode, et d'effectuer la réaction à une température allant de 25°C à 150°C.

2. Procédé selon la revendication 1, caractérisé en ce que la température de la réaction va de 70° à 100°C.

3. Procédé selon la revendication 1, caractérisé en ce que la 2-halopyridine est la 2,3-dichloro-5-(trifluorométhyl)-pyridine.

4. Procédé selon la revendication 1, caractérisé en ce que la 2-halopyridine est la 2-chloro-5-(trifluorométhyl)-pyridine.

5. Procédé selon la revendication 1, caractérisé en ce que le solvent est le diméthylsulfoxyde.

6. Procédé selon la revendication 1, caractérisé en ce que le solvent est la N-méthylpyrrolidone.

7. Procédé selon la revendication 1, caractérisé en ce que l'hydroquinone a été neturalisée, de 90 à 100 pour cent, en sel de sodium.